Europäisches Patentamt

**European Patent Office**

**Office européen des brevets**

(11) Numéro de publication: **0 127 503**
**B1**

## FASCICULE DE BREVET EUROPÉEN

(45) Date de publication du fascicule du brevet:
**12.07.89**

(51) Int. Cl.⁴: **A 61 F 2/40**

(21) Numéro de dépôt: **84400852.4**

(22) Date de dépôt: **26.04.84**

(54) **Prothèse d'épaule.**

(30) Priorité: **02.05.83 FR 8307251**

(43) Date de publication de la demande:
**05.12.84 Bulletin 84/49**

(45) Mention de la délivrance du brevet:
**12.07.89 Bulletin 89/28**

(84) Etats contractants désignés:
**BE CH DE GB IT LI LU NL**

(56) Documents cité:
**DE-A-2 344 569**
**FR-A-2 103 145**
**US-A-3 803 641**
**US-A-3 815 157**
**US-A-3 916 451**
**US-A-3 978 528**
**US-A-4 206 517**

(73) Titulaire: **Société anonyme: COMPAGNIE ORIS INDUSTRIE, 33, rue de la Fédération BP 510, F-75752 Paris Cédex (FR)**

(72) Inventeur: **Gabard, Jean- Jacques, 17 Route des Alluets "Le Bois Beullé" Bazemont, F-78580 Maule (FR)**

(74) Mandataire: **Pottier, Pierre Société BREVATOME, 25, Rue de Ponthieu, F-75008 Paris (FR)**

Il est rappelé que: Dans un délai de neuf mois à compter de la date de publication de la mention de la délivrance du brevet europeen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne une prothèse d'épaule et, plus spécialement une telle prothèse exempte du risque de luxation en toutes positions du bras, même en présence de faiblesse marquée ou de dégénérescence importante des tendons et des tissus musculaires qui normalement assurent la suspension de l'épaule sur l'omoplate.

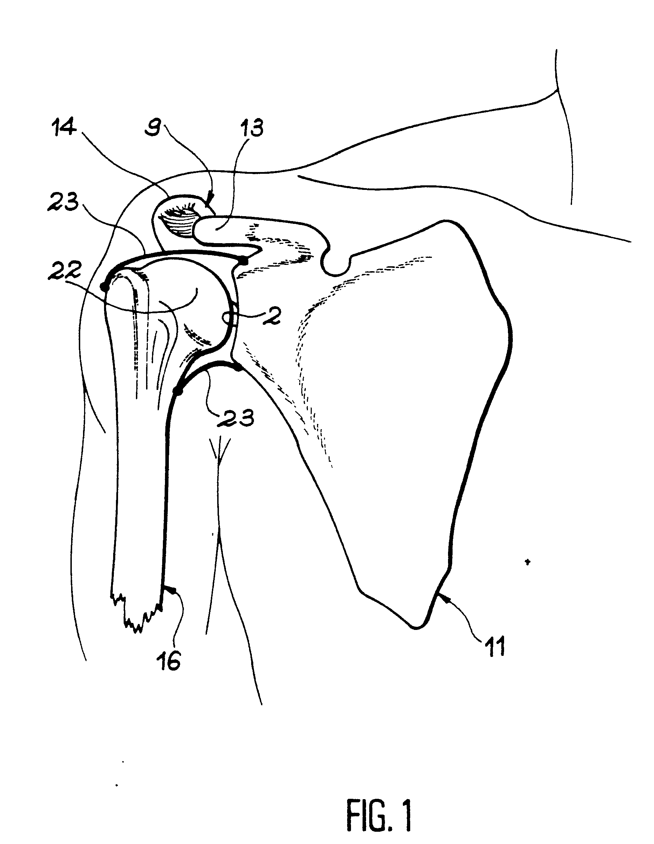

On commencera, pour bien faire comprendre l'objet de l'invention, par rappeler la conformation anatomique d'une épaule humaine normale en se référant à la description de la figure 1.

Sur cette figure 1, on a représenté une épaule humaine normale comprenant, de façon connue, une omoplate 11 et un humérus dont on voit la partie supérieure 16 et la tête humérale 22. La tête humérale 22, de forme sensiblement sphérique, vient en contact avec la glène 2 de l'omoplate 11, qui est une cavité cartilagineuse contre laquelle glisse et roule cette tête humérale 22. La particularité d'une telle articulation réside dans le fait que le maintien en place de l'humérus 16 contre l'omoplate 11 est assuré uniquement par suspension, passive à l'aide des ligaments 23, et active à l'aide des muscles périarticulaires qui relient l'humérus 16 et l'omoplate 11.

Lors de lésions dégénératives aigües ou chroniques de l'épaule ou lors de fractures de la tête, du col huméral ou de la glène de l'omoplate, il est parfois nécessaire de remplacer les parties atteintes ou fracturées par des implants prothétiques, sur l'humérus et sur l'omoplate.

Différents remplacements prothétiques de l'épaule ont déjà été proposés.

Les premiers, proposés notamment après des fractures de la tête humérale interdisant toute restauration par ostéosynthèse (vis, plaque, broches), consistaient en une pièce prothétique humérale scellée ou non avec du ciment dans le fût diaphysaire de l'humérus après que l'on ait pratiqué l'exérèse des fragments fracturés de la tête humérale.

D'autres, plus complètes, ont été ensuite proposées aux chirurgiens. Ces prothèses étaient orientées vers la réalisation d'un implant prothétique total, c'est-à-dire le remplacement de la tête humérale associé au remplacement, après exérèse, du cartilage articulaire glénoïdien (omoplate) en contact avec cette tête prothétique.

Ces différentes solutions présentent de nombreux inconvénients.

En effet, si le positionnement de la pièce glénoidienne ne pose pas de problèmes majeurs, l'orientation en rotation de la pièce humérale est délicate, en raison des conditions opératoires et du manque de repère après la résection de la tête humérale.

D'autre part, les résultats cliniques sont diminués par le fait que le tissu musculaire est souvent médiocre avant l'intervention (sujets âgés peu musclés dans le cas de fracture, ou épaule "bloquée" depuis plusieurs années en cas d'affections dégénératives) et que ce tissu musculaire est encore altéré par l'opération elle-même, qui exige de sectionner certains muscles pour réaliser une voie d'accès à l'articulation.

Enfin et surtout, en l'absence d'une liaison mécanique d'une pièce prothétique par rapport à l'autre, on aboutit soit à une épaule ballante soit à une diminution très importante des mouvements due à l'absence ou à la diminution de la synergie des muscles moteurs de cette articulation prothésée.

En effet, dans une épaule normale, la stabilité de la tête humérale est assurée comme on l'a vu sur la figure 1 par les ligaments, la capsule articulaire et les muscles périarticulaires coapteurs.

L'affection en cause, traumatique ou non, détériore certains muscles et l'intervention chirurgicale aggrave encore cette atteinte musculaire et risque de léser les ligaments et la capsule articulaire, que l'on est obligé de traverser et de réparer ensuite.

C'est pourquoi, une deuxième catégorie de prothèses d'épaule, illustrées par exemple par les brevets US-A-3 978 528 et US-A-4 206 517, a été proposée, dans lesquelles, renonçant à restaurer tant bien que mal l'épaule anatomique, on s'est résolument orienté, au contraire, vers la fabrication d'une épaule rotulante artificielle, sans rapport avec l'épaule normale. Dans de telles prothèses, en effet, une tête sphérique fixée sur l'omoplate rotule dans un logement creux sphérique ou cupule fixé sur l'humérus, dont elle prend la place de l'ancienne tête, à l'aide d'une queue diaphysaire cimentée dans l'axe de l'humérus.

Les concepteurs de telles prothèses avaient sans doute pensé, à juste titre, qu'une rotule est plus satisfaisante mécaniquement qu'un contact roulant, et se sont visiblement inspirés, comme cela ressort de US-A-4 206 517, de l'articulation de la hanche (cf. Fig. 13, 14 et 15). Toutefois, d'une part, les conditions de fonctionnement de l'épaule (en général en traction) sont très différentes de celles de la hanche (en général en compression) et, d'autre part, les possibilités de luxation des prothèses décrites dans les brevets US-A-3 978 528 et US-A-4 206 517 sont très réelles si le système des ligaments et des muscles sustentateurs est plus ou moins déficient.

En effet, dans les deux brevets précédents, la queue diaphysaire fait un angle d'environ 60° avec la surface libre de la cupule, ce qui conduit à relier cette queue diaphysaire au voisinage du sommet de la demi-sphère constituant la cupule et à la nécessité de prévoir un système mécanique intermédiaire (rondelle de plastique encliquetable 38 dans US-A-3 978 528 et association d'un boîtier rigide 14 et d'une rondelle élastique 15 dans US-A-4 206 517) pour verrouiller la sphère dans son logement et rendre la cupule rétentive, notamment dans les positions abaissées du bras. En d'autres termes, le risque de luxation de l'épaule n'est écarté que par la présence d'un tel système intermédiaire, nécessairement fragile et dégradable.

Enfin, le fait que, dans ces mêmes réalisations, l'axe de la queue diaphysaire - ou de l'humérus - ne passe pas par le centre de la sphère rotulante, crée un couple complémentaire de la force de traction du bras par rapport au centre de la rotule, ce qui fragilise encore davantage de telles conceptions.

L'ensemble des inconvénients précédents a interdit pratiquement jusqu'à ce jour la pose de telles prothèses rotulantes d'épaule qui sont demeurées au stade de projets.

La prothèse totale objet de l'invention a pour but de remédier aux inconvénients précédents et sa constitution particulière, suspendue mécaniquement à l'omoplate, permet d'éviter tout risque de luxation, même dans le cas de défaillance sérieuse des systèmes ligamentaires et/ou musculaires de soutien habituels de l'épaule normale.

Cette prothèse de l'épaule humaine suspendue mécaniquement à l'omoplate et exempte de risque de luxation en toutes positions du bras, comprenant un système rotulant composé d'une sphère fixée sur l'omoplate par l'intermédiaire d'une tige de fixation solidaire d'un implant ancré dans la glène, et d'une cupule humérale articulée sur la phère et fixée à l'humérus par une queue diaphasaire supportant la cupule humérale et implantée dans l'humérus, la surface externe de la cupule humérale étant sphérique, concentrique à la sphère creuse interne de la cupule humérale et de diamètre voisin de celui d'une tête humérale anatomique normale, se caractérise en ce que:

- l'axe de la queue diaphysaire passe par le centre de la sphère et son angle avec le plan de la surface contenant le bord de l'ouverture de la cupule humérale est voisin de 10°,
- ta sphère est montée à force sans pièce intermédiaire dans la cupule humérale qui est rétentive vis-à-vis de cette sphère qu'elle entoure au-delà d'un plan diamétral,
- l'axe de la tige de fixation de la sphère sur la glène de l'omoplate ne passe pas par le centre de cette sphère et est décalé vers le bas, l'implant étant en forme de sabot et coiffant la glène.

Selon une caractéristique secondaire de la prothèse d'épaule objet de l'invention, le sabot glénoïdien se prolonge à sa partie inférieure par une gouttière conformée pour coiffer le bord axillaire de l'omoplate, et il comporte des trous pour la mise en place de vis, lesdits trous étant placés en regard des parties dures de l'omoplate, la gouttière du sabot portant des trous pour la mise en place d'une clavette traversant le bord axillaire de l'omoplate, le sabot glénoïdien étant muni, en outre, sur sa face interne, d'une pièce de retenue en forme de queue d'aronde.

Ces caractéristiques et toutes autres, ainsi que le mode de mise en place de la prothèse selon l'invention seront décrits ci-après en détail avec référence aux dessins annexés sur lesquels:

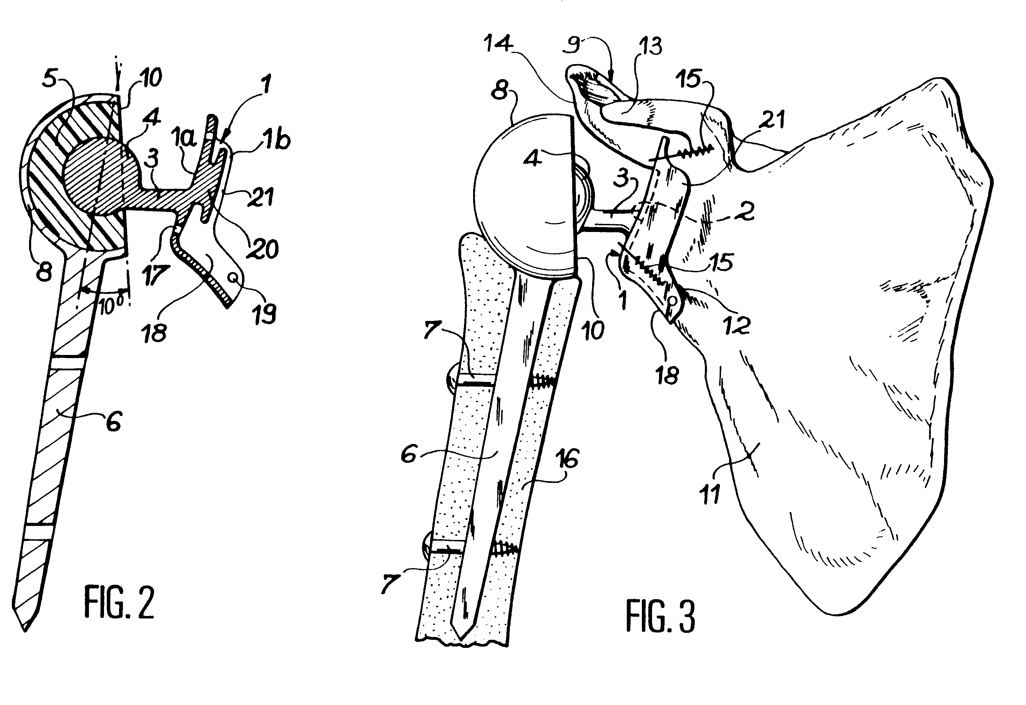

- la fig. 2 est une vue en coupe longitudinale d'un exemple de réalisation préféré de l'invention;

la figure 3 est une élévation avec coupe partielle de la prothèse de la figure 1 mise en place, l'humérus et l'omoplate étant représentés en traits fins;

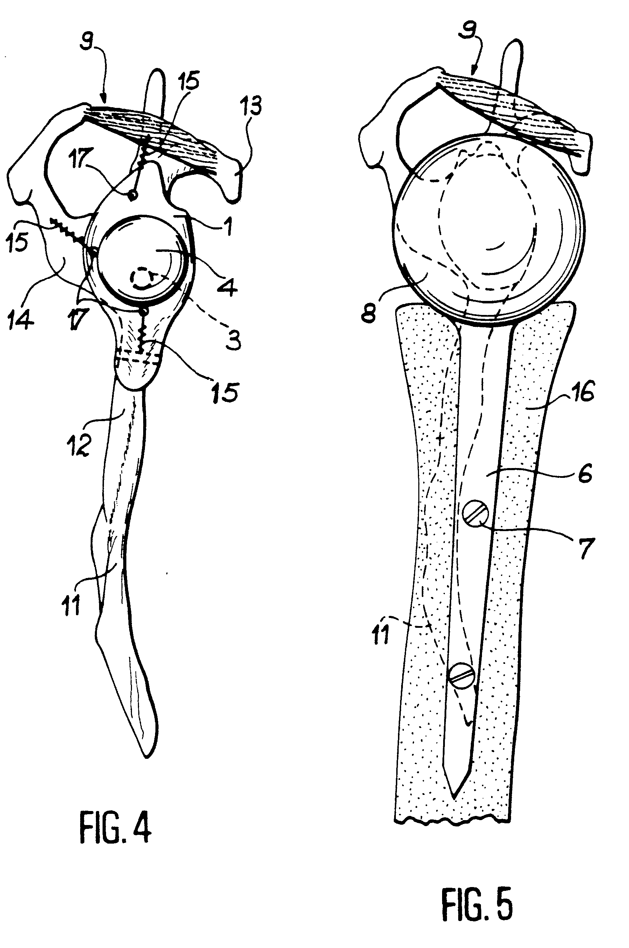

la figure 4 est une vue en bout de la partie glénoïdienne avec représentation de l'omoplate en traits fins;

la figure 5 est une vue analogue de la partie humérale de la prothèse avec représentation en traits fins de l'humérus et de l'omoplate.

En se reportant à ces figures, on voit que la prothèse totale de l'invention se compose de la combinaison d'une partie glénoïdienne, formée d'un sabot 1 coiffant la glène 2 de l'omoplate 11, portant un bras 3 terminé à son extrémité par une sphère ou boule 4, avec une partie humérale formée d'une cupule sensiblement hémisphérique 5 de même diamètre que la boule 4 et portant une queue 6 destinée à être implantée dans le fût diaphysaire de l'humérus 16.

La queue 6 est de préférence scellée dans la cavité médullaire de l'humérus avec du ciment et des vis transversales 7 ou l'équivalent sont de préférence prévues pour éviter à la fois les risques de déplacement par efforts d'arrachement et efforts en rotation.

La boule 4 pourra être métallique et la cupule 5 est tapissée intérieurement d'une matière plastique de faible coefficient de frotttement tel que le polyéthylène, mais l'invention n'est pas limitée aux matériaux employés.

La cupule 5 est de préférence rétentive, c'est-à-dire que son ouverture est de diamètre légèrement inférieur au diamètre de la boule 4, laquelle est mise en place à force, ce qui améliore sa tenue.

Le dimensionnement extérieur de cette tête prothétique est tel qu'elle doit se loger sans difficulté sous la voûte acromioclaviculaire 9 osseuse laissée en place et permettre le glissement pour l'élévation du bras ainsi que les autres mouvements.

De préférence également, la face externe 8 de la cupule 5 constituant la tête prothétique est de forme sphérique concentrique à la sphère de la cupule 5 et de diamètre voisin de celui de la tête humérale anatomique qu'elle remplace.

Cette disposition permet le mouvement d'abduction sans risque de luxation, la tête humérale 8 prenant appui sur la voûte acromio-coracoïdienne 9.

Le bord d'ouverture 10 de la cupule 5 forme un plan ou se trouve sensiblement selon un plan qui forme un angle de 10° environ avec la queue 6 (ou avec l'axe ou la direction générale de la queue 6).

Cette disposition assure une suspension positive de l'humérus pour tous les mouvements d'abduction, d'antipulsion, d'adduction et de rotation interne.

D'autre part, elle assure une liberté de rotation vers le haut de l'humérus de l'ordre de 90° avant que le bord 10 de la cupule 5 ne vienne buter contre le bras 3. A cette fin, le bras 3 est de préférence désaxé vers le bas par rapport à la sphère 4 comme représenté.

De plus, le sabot 1 étant fermement fixé sur l'omoplate (voir plus loin), la combinaison de cette disposition avec le dimensionnement externe de la tête 8 provoque un entraînement de l'omoplate 11 une fois le bord 10 en butée contre le bras 3, ce qui rétablit le mouvement de l'omoplate et permet un supplément de rotation en abduction de l'ordre de 30° sans luxation.

La longueur du bras 3 est supérieure à l'épaisseur de la cupule 5 et en pratique de l'ordre de 2 cm à 3 cm.

Le diamètre de la boule 4 sera également de l'ordre de 2 cm pour un diamètre de tête 8 de l'ordre de 4 à 5 cm.

Il est évident que ces dimensions se rapportent à une prothèse d'adulte de taille moyenne et que ces dimensions seront adaptées à différentes possibilités de tailles anatomiques, pour constituer une gamme répondant aux besoins chirurgicaux.

Le bras 3 est rigidement fixé à un sabot 1 qui épouse la forme de la glène en la coiffant.

Etant donné que le sabot 1 est destiné à être mis en place sur la surface glénoïdienne débarrassée du cartilage articulaire et préalablement creusée, 1a face 1a peut être une face plane avec des bords latéraux 1b coiffant l'os latéralement

La face 1a présente des orifices calibrés 17 placés de façon à permettre la fixation par des vis 15 pénétrant dans les parties dures de l'omoplate (bord axillaire de l'omoplate 12, massif coracoïdien 13, massif de l'épine omoplate 14).

L'ancrage du sabot 1 sur la glène 2 de l'omoplate 11 est complété par les moyens suivants considérés séparément ou en combinaison.

- Le sabot 1 porte sur sa partie inférieure une gouttière 18 coiffant le bord axillaire de l'omoplate 11 et il est prévu une clavette 19 reliant les bords de cette gouttière en traversant ledit bord axillaire.

Le sabot 1 porte sur sa face interne une pièce 20 en forme de queue d'aronde, c'est-à-dire ayant une tête 21 plus étendue que sa base.

En vue de la mise en place de la prothèse, la glène est préalablement débarrassée de cartilage et creusée dans toute la partie spongieuse, le sabot 1 est ensuite mis en place et vissé avec injection de ciment entre la zone creusée et le sabot qui se trouve ainsi fermement rattaché à l'omoplate et peut entraîner celle-ci en rotation à la fin des mouvements d'abduction du bras.

Il a été indiqué plus haut que les différentes parties de la prothèse selon l'invention seront réalisées en tous matériaux. Différents métaux peuvent être utilisés tels que l'acier, le titane, poreux ou non, ainsi que différentes matières plastiques ou les bio-matériaux, par exemple carbonés, permettant ou non la réhabilitation osseuse à l'intérieur de la surface de certains éléments.

L'invention n'est pas non plus limitée à l'exemple de réalisation décrit, différentes variantes de formes et de dimensions pouvant être adoptées sans sortir du cadre des revendications.

## Revendications

1. Prothèse de l'épaule humaine suspendue mécaniquement à l'omoplate (11) et exempte de risque de luxation en toutes positions du bras, du type comprenant un système rotulant composé d'une sphère (4) fixée sur l'omoplate (11) par l'intermédiaire d'une tige de fixation (3) solidaire d'un implant (1) ancré dans la glène, et d'une cupule humérale (5) articulée sur la sphère (4) et fixée à l'humérus par une queue diaphysaire (6) supportant la cupule humérale (5) et implantée dans l'humérus (16), la surface externe (8) de la cupule humérale (5) étant sphérique, concentrique à la sphère creuse interne de la cupule humérale (5) et de diamètre voisin de celui d'une tête humérale anatomique normale, caractérisée en ce que:

- l'axe de la queue diaphysaire (6) passe par le centre de la sphère (4) et son angle avec le plan de la surface contenant le bord de l'ouverture (10) de la cupule humerale (5) est voisin de 10°,
- la sphère (4) est montée à force sans pièce intermédiaire dans la cupule humérale (5) qui est rétentive vis-à-vis de cette sphère qu'elle entoure au-delà d'un plan diamétral,
- l'axe de la tige de fixation (3) de la sphère (4) sur la glène (2) de l'omoplate (11) ne passe pas par le centre de cette sphère et est décalé vers le bas, l'implant (1) étant en forme de sabot et coiffant la glène.

2. Prothèse selon la revendication 1, caractérisée en ce que le sabot glénoïdien (1) se prolonge à sa partie inférieure par une gouttière (18) conformée pour coiffer le bord axillaire de l'omoplate (11), et qu'il comporte des trous (17) pour la mise en place de vis (15), lesdits trous (17) étant placés en regard des parties dures de l'omoplate (11), la gouttière (18) du sabot (1) portant des trous pour la mise en place d'une clavette (19) traversant le bord axillaire de l'omoplate (11), le sabot glénoïdien (1) étant muni, en outre, sur sa face interne, d'une pièce de retenue (20) en forme de queue d'aronde.

## Patentansprüche

1. Menschliche Schulterprothese, die mechanisch am Schulterblatt (11) aufgehängt ist und in allen Positionen des Armes gegen die Gefahr einer Luxation sicher ist, von jener Art, die ein Kugelsystem enthält, bestehend aus einer Kugel (4), die am Schulterblatt (11) mittels eines Haltearmes (3) befestigt ist, der fest mit einem Einsatz (1) verbunden ist, der in der Gelenkpfanne verankert ist, und aus einer Oberarmglocke (5),

die auf der Kugel (4) gelenkig gelagert und am Oberarm mittels eines Knochennagels (6) befestigt ist, die Oberarmglocke (5) trägt und in den Oberarm (16) implantiert ist, wobei die Außenfläche (8) der Oberarmglocke (5) kugelförmig und konzentrisch zu dem kugelförmigen hohlraum der Oberamglocke (5) ist und deren Durchmesser nahezu dem des normalen anatomischen Oberarmkopfes gleich ist, dadurch gekennzeichnet,

daß die Achse des Knochennagels (6) durch das Zentrum der Kugel (4) verläuft und daß sein Winkel mit der Ebene jener Fläche, in der der Rand der Öffnung (10) der Oberarmglocke (5) liegt, etwa 10° beträgt,

die Kugel (4) fest ohne Zwischenstück in der Oberarmglocke (5) gehalten ist, die gegenüber dieser von ihr umgebenen Kugel jenseits einer Diametralebene gehalten ist,

die Achse des Haltearmes (3) der Kugel (4) auf der Gelenkpfanne (2) des Schulterblattes (11) nicht durch die Mitte dieser Kugel verläuft und nach unten versetzt ist, wobei der Einsatz (1) die Form eines Schuhs hat und die Gelenkpfanne überdeckt.

2. Prothese nach Anspruch 1, dadurch gekennzeichnet, daß der Gelenkpfannenschuh (1) sich in seinem unteren Abschnitt durch eine Rinne (18) fortsetzt, die so gestaltet ist, daß sie den Achselrand des Schulterblattes (11) bedeckt, und daß sie Löcher (17) für die Anbringung von Schrauben (15) aufweist, die gegenüber den harten Teilen des Schulterblattes (11) angeordnet sind, wobei die Rinne (18) des Schuhs (1) Löcher für die Anbringung eines Ankers aufweist, der durch den Achselrand des Schulterblattes (11) verläuft, und der Gelenkpfannenschuh (1) darüberhinaus auf seiner Innenseite mit einem Haltestück (20) von Schwalbenschwanz-förmiger Gestalt versehen ist.

## Claims

1. Human shoulder prosthesis suspended mechanically on the scapula (11) and free from risk of dislocation in all positions of the arm, of the type comprising a ball-and-socket system consisting of a sphere (4) fixed on the scapula (11) by means of a fixation rod (3) integral with an implant (1) anchored in the glene, and of a humeral cup (5) articulated on the sphere (4) and fixed to the humerus by a diaphyseal shaft (6) supporting the humeral cup (5) and implanted in the humerus (16), the outer surface (8) of the humeral cup (5) being spherical, concentric to the hollow internal sphere of the humeral cup (5) and of a diameter close to that of a normal anatomical humeral head, characterized in that:

-the axis of the diaphyseal shaft (6) passes through the centre of the sphere (4), and its angle to the plane of the surface containing the edge of the opening (10) of the humeral cup (5) is around 10°,

-the sphere (4) is driven, without any intermediate component, into the humeral cup (5) which is retentive relative to this sphere which it encloses beyond a diametral plane,

-the axis of the fixation rod (3) of the sphere (4) on the glene (2) of the scapula (11) does not pass through the centre of this sphere and is offset towards the bottom, the implant (1) being in the form of a saddle and covering the glene.

2. Prosthesis according to claim 1, characterized in that the glenoid saddle (1) is extended, at its lower part, by a trough (18) designed to cover the axillary edge of the scapula (11), and in that it comprises holes (17) for the positioning of screws (15), the said holes (17) being placed opposite the hard areas of the scapula (11), the trough (18) of the saddle (1) having holes for the positioning of a pin (19) passing through the axillary edge of the scapula (11), the glenoid saddle (1) being additionally equipped, on its inner face, with a retention piece (20) in the form of a dovetail.

FIG. 1

FIG. 2

FIG. 3

EP 0 127 503 B1

FIG. 4

FIG. 5